# NEUE EUROPÄISCHE PATENTSCHRIFT

(11) **EP 0 917 430 B2**
(45) Veröffentlichungstag und Bekanntmachung des Hinweises auf die Entscheidung über den Einspruch: **12.03.2008**
(45) Hinweis auf die Patenterteilung: 19.02.2003
(21) Anmeldenummer: 97922981.2
(22) Anmeldetag: 06.05.1997
(51) Int. Cl.: A23K 1/00, C12N 9/98

(54) **ENZYM-VORGRANULAT FÜR TIERFUTTERMITTEL-GRANULATE**
ENZYME PRE-GRANULES FOR GRANULAR FODDER
PREGRANULE ENZYMATIQUE POUR FOURRAGE SOUS FORME DE GRANULES

(30) Priorität: 13.05.1996 DE 19619219
(43) Veröffentlichungstag der Anmeldung: 26.05.1999
(73) Patentinhaber: Biovet JSC, 4550 Peshtera (BG)
(72) Erfinder: MESCHONAT, Beate, D-30419 Hannover (DE); HERMANN, Hubert, D-38162 Cremlingen (DE); SPANNAGEL, Rolf, D-32629 Husum (DE); SANDER, Vera, D-30826 Garbsen (DE); KONIECZNY-JANDA, Gerhard, D-30982 Pattensen (DE); SOMMER, Mario, D-61389 Schmitten (DE)
(74) Vertreter: Leifert & Steffan
(86) Internationale Anmeldenummer: PCT/EP1997/002306
(87) Internationale Veröffentlichungsnummer: WO 1997/042837

(56) Entgegenhaltungen:
- EP-A- 0 257 996
- EP-A- 0 280 226
- EP-A- 0 516 867
- EP-A- 0 520 890
- WO-A-92/11347
- WO-A-92/12645
- WO-A-94/23005
- WO-A-95/18544
- WO-A-97/12958
- WO-A-97/16076
- WO-A-97/42839
- PATENT ABSTRACTS OF JAPAN vol. 012, no. 155 (C-494), 12.Mai 1988 & JP 62 269685 A (SHOWA DENKO KK), 24.November 1987,
- Ullmann's Encyclopedia of Industrial Chemistry, Bd. A11, S. 551, 552, 1988
- Heimann, Grundzuege der Lebensmittelchemie, 1976, S. 361-364

## Beschreibung

Die Erfindung betrifft die Herstellung eines aktivitätsstabilen Enzym-Vorgranulates, das dem Zweck dient, in Teilchen eines Tierfuttermittel-Granulates eingearbeitet zu werden. Weiterhin betrifft die Erfindung die nach den Herstellverfahren erhaltenen aktivitätsstabilen Vorgranulate zur Einarbeitung in Tierfuttermittel-Granulate.

Der Einsatz von Enzymen in Tierfuttermitteln ermöglicht eine bessere Verwertbarkeit der im Tierfutter enthaltenen Nährstoffe, da durch den Zusatz von Enzymen die Verwertung von nicht vollständig oder schwer verdaulichen Bestandteilen für das Tier erleichtert wird. Die Enzym-Zugabe zum Tierfuttermittel erweist sich hierbei als ein wirkungsvolles Mittel, um Futtermittel in unkonventioneller und preiswerter Zubereitung zu entwickeln, die eine optimale Ausnutzung der im Futtermittel enthaltenen Nährstoffe gewährleisten. Damit wird es möglich. preiswerte Rohmaterialien wie Getreide; Bohnen oder andere Samen in optimaler Weise für die Herstellung von qualitativ hochwertigen Futtermittelkomponenten, die insbesondere auch für Jungtiere geeignet sind, zu verwenden. Für die Anwendung von Enzymen in Tierfuttermitteln steht eine umfangreiche Anzahl von Enzymen zur Verfügung, die spezielle Enzymaktivitäten zum Abbau von speziellen Futtermittelbestandteilen wie z.B. Glukane, Stärke, Proteine, pektinähnliche Polysaccharide, Phytinsäure, Galactomannane, Galactoarabane, Polygalacturone, Raffinose, Stachyose, Hemicellulose, Cellulose, Pentosane und andere Nährbestandteile aufweisen. Tierfuttermittel liegen-häufig als Granulate, d.h. als gröbere oder körnige Haufwerke vor; der Tierfuttermittel-Fachmann spricht dann oft von "Futtermittel-Pellets", auch wenn die Form der Granulat-Teilchen nicht im eigentlichen Sinne des Wortes "Pellet" (kugelförmiges Teilchen) ausgerundet ist. Die Enzyme können hierbei dem Futtermittel, einer Vormischung desselben oder auch einem Vermischungsbestandteil zugemischt oder aber in Granulate dieser Bestandteile eingearbeitet werden. Granulierte Enzym-Produkte können sehr leicht mit den Futtermittelkomponenten vermischt werden, soweit diese Enzym-Granulate auf gewöhnlichen Futtermittelkomponenten basieren, wie z.B. Weizen oder Sojaflocken. Im Stand der Technik sind auch Verfahren bekannt, die gelöste Enzyme auf Puttermittelteilchen in einem Fließbett, in einem bewegten oder gerührten Bett aufsprühen und nachfolgend gegebenenfalls trocknen. Nachteilig ist hierbei, daß oftmals eine unerwünschte Verkeimung des Futtermittels beobachtet wird. Weiterhin kann es erwünscht sein, die Enzyme direkt in die Teilchen eines Tierfuttermittel-Granulates einzuarbeiten, um eine möglichst intensive und homogene Vermischung des Enzyms in den Teilchen des Tierfuttermittel-Granulates selbst zu erzielen. Bei der Herstellung eines solchen Futtermittel-Granulates werden daher die gewünschten Bestandteile des Tierfuttermittels mit einer Enzymzubereitung gemischt, die Mischung nachfolgend durch Bedüsen mit Dampf konditioniert und anschließend daraus über Extrusion Strangschnittlinge erzeugt. Bei der Herstellung solcher Tierfuttermittel-Granulate werden die zugesetzten Enzyme jedoch einer hohen Temperaturbeanspruchung und einer hohen Druckbeanspruchung und Reibungs- und Scherkräften ausgesetzt. Dadurch wird die ursprüngliche Enzymaktivität oftmals nicht nur sehr beeinträchtigt, sondern geht oftmals sogar vollständig verloren.

Es bestand daher die Aufgabe, eine geeignete Verfahrensweise zur Formulierung von Futtermittelenzymen bereitzustellen, um die vorstehenden Nachteile zu beheben und um es zu ermöglichen, die Enzyme ohne wesentliche Aktivitätsverluste in die Teilchen eines Tierfuttermittel-Granulates homogen einzuarbeiten.

Die Aufgabe wird gelöst durch das im Anspruch 1 angegebene Verfahren sowie durch die nach diesen Verfahren hergestellten aktivitatsstabilen Enzym-Vorgranulte gemäß Anspruch 16 sowie die im Anspruch 18 angegebene Verwendung. Zweckmäßige Ausgestaltungen des erfindungsgemäßen Verfahrens sind in den Unteransprüchen 2 bis 15 und im Hinblick auf das erfindungsgemäße Enzym-Vorgranulat im Unteranspruch 17 wiedergegeben.

Demgemäß wird durch die Erfindung ein Verfahren zur Herstellung eines aktivitätsstabilen Enzym-Vorgranulates bereitgestellt, welches zur Einarbeitung in die Teilchen eines Tierfuttermittel-Granulates dient, wobei sich das erfindungsgemäße Verfahren dadurch auszeichnet, daß man zunächst eine Feuchtgranulat herstellt, indem man
0,01 bis 20 Gew.-Teile Enzym oder Enzymgemisch (berechnet als Trockensubstanzgehalt des eingesetzten Enzympräparates),
80 bis 99,99 Gew.-Teile (inklusive Feuchtegehalt) einer organischen Mehltype mit einem Ausmahlungsgrad von 30 % bis 100 %,
wobei die Mehltype durch Ausmahlen einer, gegebenenfalls zuvor gewaschenen und/oder gereinigten, mit trockenem Heißdampf behandelten Mehlquelle gewonnen wurde,
und wobei sich die Gew.-Teile des Enzyms oder Enzymgemisches und der Mehltype auf 100 Gew.-Teile summieren,
gewünschtenfalls bis zu insgesamt 20 Gew.-Teile Granulierhilfsstoffe (berechnet als wasserfreie Granulierhilfsstoffe),
unter Verwendung einer berechneten Menge Wasser, die zur Einstellung eines Feuchtegehaltes im Feuchtgranulat von 20 bis 50 Gew.-% (bezogen auf die Summe der Bestandteile des Feuchtgranulates als 100 Gew.-%) ausreicht,
in einem Schnellmischer durch intensives Mischen zu einem klebefreien Feuchtgranulat mit gewünschtem Korngrößenbereich aufbaut,
man das derart erhaltene Feuchtgranulat trocknet und nachfolgend gewünschtenfalls noch durch Siebung von Unter- und/oder Überkorn befreit. Der Begriff "klebefrei" bedeutet hierbei, daß das Feuchtgranulat nicht mehr an den Mischeinrichtungen oder der Mischerwand anhaftet.

In einer zweckmäßigen Ausgestaltung der Erfindung zeichnet sich das vorstehende Verfahren dadurch aus, daß man zur Herstellung des Feuchtgranulates
0,01 bis 10 Gew.-Teile Enzym oder Enzymgemisch, vorzugsweise
2 bis 7 Gew.-Teile Enzym oder Enzymgemisch,
90 bis 99,99 Gew.-Teile Mehltype, vorzugsweise 93 bis
98 Gew.-Teile Mehltype,
gewünschtenfalls bis zu insgesamt 15 Gew.-Teile, vorzugsweise 0,5 bis 5 Gew.-Teile, Granulierhilfsstoffe,
und eine berechnete Menge Wasser einsetzt, die zur Einstellung eines Feuchtegehaltes von 25 bis 40 Gew.-%, vorzugsweise von 25 bis 35 Gew.-%, im Feuchtgranulat ausreicht.

Gemäß der Erfindung werden organische Mehle (also Mehle aus organischen Grundstoffen) bestimmten Types eingesetzt. Die Bezeichnung "organisches Mehl" umfaßt hierbei im Rahmen der Erfindung alle mehr oder weniger zerkleinerten, pulverförmigen bis feinkörnigen Produkte, die durch Zerkleinern (Mahlen) aus festen organischen Materialien natürlichen Ursprungs (Mehlquelle) gewonnen wurden. Zweckmäßigerweise werden im erfindungsgemäßen Verfahren organische Mehle eingesetzt, die man durch Mahlen von Getreidekörnern, Leguminosenfrüchten und/oder Früchten der Familie Malvaceae (z.B. Baumwollsamen) erhält. Die vorzugsweise im Rahmen der Erfindung als Mehlquelle dienenden Getreide sind insbesondere Weizen oder Roggen, aber auch Gerste, Hafer, Reis und Mais sowie Sorghum und andere Hirse-Arten können eingesetzt werden. Obwohl Buchweizen an sich nicht zu den Getreidearten gehört (Knöterichgewächs), können dessen bucheckerähnliche Mehlfrüchte ebenfalls im Rahmen der Erfindung als Mehlquelle eingesetzt werden; dies gilt insbesondere im Hinblick für Tierfuttermittel-Granulate für Geflügel, jedoch ist bei der Herstellung von Tierfuttermittel-Granulaten für Weidevieh wegen des Gehaltes an photosensibilisierendem Fagopyrin gegebenenfalls Vorsicht geboten, soweit eine Verfütterung an überwiegend weiß behaartes Vieh beabsichtigt ist. In einer anderen bevorzugten Variante der Erfindung dienen Leguminosenfrüchte als Mehlquelle. Unter Leguminosen werden hier die zu den Fruchtgemüsen gehörenden, pflanzlichen Nahrungsmittel (Hülsenfrüchte) verstanden. Als Mehlquelle im Rahmen der Erfindung kommen daher die Früchte der Leguminosen-Arten wie: Pisum (Erbsen), Cajamus (Straucherbse), Cicer (Kichererbse); Lens (Linsen); Phaseolus (Bohnen), Vigna (Kuhbohnen), Dolchius (Helmbohnen), Cassavalia (Schwercbohnen), Vicia (Pferdebohnen oder Wicken); Peluschken; Arachis (Erdnüsse); Lupinen; Luzerne; Sojabohnen sowie Limabohnen und ggf. andere Hülsenfrüchte und auch Malvaceae-Früchte (z.B. der Gattung Gossipium, Baumwolle) in Betracht. Bevorzugt sind Sojabohnen.

Von ölhaltigen Früchten der vorstehenden Arten können sowohl entölte, teilentölte als auch ölhaltige Früchte zur Gewinnung des erfindungsgemäß verwendeten Mehls eingesetzt werden; bevorzugt sind hierfür teilentölte Früchte, insbesondere teilentölte Leguminosen-Früchte, z.B. teilentölte Sojabohnen.

Die im Rahmen der Erfindung einsetzbaren Mehle sind je nach den angewandten Mahlverfahren und dem dabei jeweils erzielten Ausmahlungsgrad feine Pulver von gelblich weißer bis grau-dunkler Farbe (helle bzw. dunkle Mehle) oder gegebenenfalls noch mehr oder weniger körnige (Schrot, Grieß, Feingrieß) oder weiß-gelblichbraun-melierte Erzeugnisse. Die erfindungsgemäß eingesetzten organischen Mehltypen weisen gewöhnlich einen Feuchtegehalt bis zu etwa 15 Gew.-% (z.B. einen Feuchtegehalt von 7 bis 15 Gew.-%) auf, der bei der Berechnung des prozentualen Feuchtegehaltes des erfindungsgemäß im Schnellmischer hergestellten Feuchtgranulates zu berücksichtigen ist. Üblicherweise werden in der Erfindung solche Getreidemehle eingesetzt, die einen Feuchtegehalt von etwa 10 bis 15 Gew.-%, insbesondere 13 bis 15 Gew.-% aufweisen; die Mehle aus Leguminosenfrüchten bzw. Früchten der Familie Malvaceae weisen üblicherweise einen Feuchtegehalt von etwa 9 (+/- 2) Gew.-% auf.

Weitere wichtigte Kriterien zur Charakterisierung des erfindungsmäßig eingesetzten Mehltyps sind der Ausmahlungsgrad und die sogenannte Mehltype; diese Kriterien korrelieren derart miteinander, daß mit zunehmendem Ausmahlungsgrad auch die Kennzahl der Mehltype zunimmt (d.h. des Zerkleinerungsgrades bzw. der Feinheit des Mehls). Der Ausmahlungsgrad entspricht der Gewichtsmenge des gewonnenen Mehls bezogen auf 100 Gewichtsteile des eingesetzten Mahlgutes (im Rahmen der Erfindung also des eingesetztes Getreides bzw. der Leguminosenfrüchte); er ist also eine prozentuale Mehlausbeute. Beim Mahlen des Mehls fällt in der Hauptsache zunächst reines, feinstes Mehl, z.B. aus dem Inneren des Getreidekorns, an und beim weiteren Vermahlen, also z.B. mit steigendem Ausmahlungsgrad, nimmt der Anteil an Rohfaser- und Schalengehalt im Mehl zu; der Stärkeanteil wird dabei geringer. Der Ausmahlungsgrad spiegelt sich daher auch in der sogenannten "Mehltype" wider, die als Zahlenangabe zur Klassifizierung von Mehlen - insbesondere von Getreidemehlen - verwendet wird und die auf dem Aschegehalt des Mehles beruht (sogenannte Ascheskala). Die Mehltype bzw. die Typenzahl gibt hierbei die Menge Asche (Mineralstoffe) in mg an, die beim Verbrennen von 100 g Mehltrockensubstanz zurück bleibt. Am Beispiel von Getreidemehlen kann die Typzahl wie folgt verdeutlicht werden: Je höher die Typzahl, desto dunkler ist das Mehl und desto höher ist der Ausmahlungsgrad, da der Kern des Getreidekorns in etwa nur 0,4 Gew.-%, die Schale dagegen etwa rund 5 Gew.-% Asche enthält. Ein Weizenmehl der Type 405 enthält so z.B. im Durchschnitt 0,405 Gew.-% Asche. Bei niederem Ausmahlungsgrad bestehen die Getreidemehle dagegen überwiegend aus dem zerkleinerten Mehlkörper, d.h. dem Stärkebestandteil der Getreidekörner; bei höherem Ausmahlungsgrad enthalten die Getreidemehle auch die zerkleinerte, eiweißhaltige Aleuronschicht der Getreidekörner, bei Schrot auch die Bestandteile des eiweiß- und fetthaltigen Keimlings sowie der rohfaser- und aschehaltigen Samenschalen.

Der Ausmahlungsgrad des erfindungsgemäß eingesetzten Mehls beträgt 30 % bis 100 %. Der Ausmahlungsgrad 30 % entspricht einem feinsten Mehl, der Ausmahlungsgrad 100 % einem Vollkornmehl. In zweckmäßigen Varianten des erfindungsgemäßen Verfahren zeichnet sich dieses dadurch aus, daß der Ausmahlungsgrad der Mehltype 50 % bis 100 %, vorzugsweise 70 % bis 100 % beträgt.

Das im erfindungsgemäßen Verfahren eingesetzte Mehl zeichnet sich dadurch aus, daß es aus einer Mehlquelle gewonnen wurde, die vor dem Vermahlen einer Behandlung mit trockenem Heißdampf mit einer Temperatur von insbesondere 100 bis etwa 110 °C unter annähernd Normaldruck bis geringem Überdruck (z.B. 0,8 bis 1.2 bar Überdruck) und einer Behandlungsdauer (Verweildauer in der nachfolgend beschriebenen Heißdampfbehandlungsvorrichtung) von bis zu etwa 1 Stunde unterworfen wurde. Trockener Heißdampf ist ein überhitzter und ungesättigter Wasserdampf, der in konventioneller Weise durch Überhitzung und Trennung von eventuellem Wasserkondensat oder durch Entspannen von Dampf von hohem Druck erzeugt werden kann. Die Heißdampfbehandlung der Mehiquelle kann z.B unter Verwendung eines sich nach unten verbreiternden, konischen Bunkers erfolgen, der mit einer oder mehreren Ringdüsen bzw. Dampflanzen für die Einleitung des trockenen Heißdampfes ausgestattet ist. Der Bunker kann im kontinuierlichen Betrieb mit der Mehlquelle, z.B. über Förderschnecken beschichtet und über beheizte Förderschnecken entleert werden. Die Heißdampf-behandelte Mehlquelle wird nachfolgend, z.B. in einem nachgeschalteten Fließbett-Trockner, auf einen konstanten Wassergehalt von höchstens 15 Gew.-% konditioniert und in einem weiteren Fließbett-Trockner für die nachfolgende Mahlung abgekühlt. Die behandelte, abgekühlte Mehlquelle wird danach kontinuierlich einer Vermahlmaschine zugeführt und zu einer Korngrößenverteilung mit Hauptanteil der Korngrößen im Bereich von 500 bis 50 µm ausgemahlen; vorzugsweise überschreitet der Anteil der Teilchen mit Korngrößen von kleiner 50 µm im ausgemahlenen Mehl 35 Gew.-% nicht und der Anteil von Teilchen mit Korngrößen von 300 bis 500 µm überschreitet darin 10 Gew.-% nicht. Bei einer zweckmäßigen Korngrößenverteilung beträgt der Anteil von Teilchen ≥ 300 µm höchstens 5 Gew.-%, von Teilchen im Bereich 300 µm bis 50 µm 65 bis 80 Gew.-% und von Teilchen unter 50 µm höchstens 30 Gew.-%.

Das Mischen und Granulieren der Bestandteile kann im erfindungsgemäßen Verfahren in einem satzweise arbeitenden Schnellmischer, z.B. Typ Pflugscharmischer, oder in einem kontinuierlich arbeitenden Schnellmischer, z.B. des Typs Schugi Flexomix (Fabrikat der Firma Schugi Process Engineers in Lelystadt/NL), erfolgen. Hierbei wird ein klebfreies Feuchtgranulat erhalten, indem man Wasser, ggf. über eine Enzymlösung oder mit einem eventuell zugesetzten Granulierhilfsmittel, kontinuierlich in Abhängigkeit der Zufuhr der festen Hauptbestandteile so zudosiert, daß der Feuchtegehalt im Feuchtgranulat (also vor dem Trocknen) am Auslauf des Mischers im allgemeinen 20 bis 50 Gew.-%, vorzugsweise 25 bis 40 Gew.-% und insbesondere 25 bis 35 Gew.-%, beträgt. Im erfindungsgemäßen Verfahren wird ein Feuchtgranulat mit einem Korngrößenbereich von 50 bis 800 µm, vorzugsweise 100 bis 800 µm, insbesondere von 100 bis 500 µm, aufgebaut. Die Mischzeit im Schnellmischer, bzw. bei kontinuierlicher Arbeitsweise die mittlere Verweildauer, beträgt beim erfindungsgemäßen Verfahren in der Regel bis zu maximal 15 Minuten; der Fachmann kann hierbei die Mischzeit bzw. Verweildauer an die erwünschten Eigenschaften des Feuchtgranulates (z.B. Klebfreiheit, Korngrößen) bzw. den jeweiligen Mischer anpassen. Als zweckmäßige Misch- bzw. Verweilzeiten bei satzweiser Granulation haben sich Zeiträume von etwa 2 Minuten bis 10 Minuten, insbesondere 3 bis 8 Minuten als ausreichend erwiesen. Bei kontinuierlicher Arbeitsweise sind auch wesentlich kürzere mittlere Verweilzeiten im Mischer ausreichend; so genügt bei kontinuierlicher Arbeitsweise im Schnellmischer des Typs Schugi Flexomix die von der Apparategröße und vom Mengenstrom abhängige mittlere Verweildauer nur im Bereich einiger Sekunden. z.B. bis 30 Sekunden, insbesondere 1 bis 10 Sekunden. Im Anschluß an die Granulierung wird das Feuchtgranulat einer konventionellen, enzymschonenden Trocknung. z.B. in einem Fließbett-Trockner, unterworfen und zu einem Granulat mit einem gewünschten Feuchtegehalt, insbesondere einem Feuchtgehalt von 3 bis 12 Gew.-%, vorzugsweise 7 bis 9 Gew.-% getrocknet. Nach dem Trocknen kann das Enzym-Vorgranulat noch in an sich üblicher Weise mit einem Lack oder einer Beschichtung überzogen werden. Die Beschichtung bzw. der Lack kann ein weiteres Enzym enthalten oder aber zur Färbung des Granulates dienen, bzw. auch eine Retardierung der Freisetzung des Enzyms oder Enzymgemisches, z.B. auch pH-abhängig gesteuerte Freisetzung in unterschiedlichen Magen-Darm-Bereichen, bewirken. Der Lack bzw. die Beschichtung kann hierbei sowohl kontinuierlich als auch satzweise auf das Enzym-Vorgranulat aufgebracht werden.

Im erfindungsgemäßen Verfahren können an sich alle Enzyme eingesetzt werden, die auf die Verwertbarkeit bzw. Verdaubarkeit von Nährbestandteilen in Tierfuttermitteln einen günstigen Einfluß ausüben. Das Enzym kann hierbei ein isoliertes, reines Enzym (d.h. ohne Nebenakcivicäten) oder ein Gemisch von Enzymen sein. Ein Enzymgemisch kann aus reinen Enzymen ohne Nebenaktivitäten zusammengestellt werden oder aber in einfacher Weise gleich in Form eines prozeßbedingt bei der Enzymgewinnung aus Mikroorganismen anfallenden Enzymgemisches erhalten werden; solche prozeßbedingt in Abhängigkeit vom Mikroorganismus anfallenden Enzymgemische umfassen in der Regel neben einem Hauptenzym verschiedene Begieitenzyme (sog. Nebenaktivitäten), die in der Regel eine günstige synergistische Nebenwirkung entfalten. Das Enzym oder Enzymgemisch kann somit allgemein eine Hydrolase. vorzugsweise aus der Gruppe der Carbohydrasen, Proteasen, Lipasen und Esterasen, oder eine Oxinitrilase, Tannase. Chitinase, Keratinase, Oxidase oder ein Gemisch dieser Enzyme sein. Die Carbohydrasen für das erfindungsgemäße Verfahren sind z. B. ausgewählt aus Beta-Glucanasen, Cellulasen, Amylasen, Pentosanasen (z.B. Endopentosanasen), Pectinasen, Xylanasen. Im Rahmen der Erfindung können auch andere Futtermittelenzyme, z.B. Arabanasen, Hemicellulasen, Galactomannasen, Polygalacturonasen, Phytasen, Glucoamylasen, β-Galactosidasen, Pullulanasen, Driselase^{R} und andere wie Lysozym oder Muramidasen eingesetzt werden. werden Oxidasen eingesetzt, so können diese Glucoseoxidasen oder Peroxidasen sein. Der Anteil (die Menge) des eingebrachten Enzyms hängt hierbei von der individuellen, spezifischen Enzymaktivität und der gewünschten Aktivität im fertigen Enzym-Vorgranulat ab. Beispielsweise besitzt Pentosanase in der Regel eine hohe spezifische Aktivität und kann schon in Mengen von 0,01 bis 0,1 Gew.-Teilen eine ausreichende Enzymaktivität im fertigen Enzym-Vorgranulat sicherstellen. Zur Herstellung der Enzyme bzw. Enzymgemische kommen allgemein Bakterien, speziell aus der Gattung Pseudomonas oder Bacillus, oder Fungi, speziell aus der Gattung Aspergillus, Trichoderma, Rhizopus, Penicillium, Irpex zum Einsatz. Es ist auch möglich, gewünschtenfalls die Strukturgene der Enzyme in geeignete Stämme von Mikroorganismen zu klonieren und zu exprimieren. Hierzu ist an sich jeder Mikroorganismus geeignet, der die zu klonierende DNA für das Enzym plasmidisch (episomal) oder genomisch (chromosomal) aufnimmt und die entsprechenden Funktionen ausüben kann.

Das im erfindungsgemäßen Verfahren eingesetzte Enzym oder Enzymgemisch kann in Form eines Pulvers oder einer wäßrigen Lösung des Enzyms oder Enzymgemisches verwendet werden. Zweckmäßige Enzyme bzw. Enzymgemische sind hierbei Enzympräparate, wie sie üblicherweise bei der industriellen Herstellung anfallen. Solche Enzympräparate enthalten in der Regel nicht nur ein einziges Enzym oder ein Gemisch von Enzymen, sondern noch andere herstellungsbedingte Begleitstoffe in untergeordneten Mengen. Ein Beispiel für solche Begleitstoffe sind z.B. Salze, die zur Präzipitation des Enzyms aus der Mutterlauge, wie diese nach Abtrennung der Biomasse aus einer Fermentationsbrühe erhalten wird, zugesetzt werden und bei der Präzipitation teilweise vom Enzym-Präzipitat eingeschlossen werden können. Die Enzyme oder Enzymgemische können ferner übliche Enzymstabilisatoren und übliche Stell- und Konservierungsmittel als weitere Begleitstoffe enthalten. Beispiele für solche Begleitstoffe sind Alkohole, Glykole, Glykolether, wie 1-Methyloxy-2-propanol, Isopropanol, Butyldiglykol, Natriumbenzoat, Calciumsalze, Glukose, Parabene, Kalium- und Natriumsorbat, Kochsalz. Werden wäßrige Lösungen des Enzyms oder Enzymgemisches eingesetzt, so können diese durch nachträgliches Lösen von Enzym- bzw. Enzymgemisch-Pulvern zubereitet werden; oder es können in einer anderen Variante auch direkt die Mutterlaugen, wie diese nach Abtrennung der Biomasse aus der Fermentabionslösung anfallen, gegebenenfalls nach Aufkonzentrierung oder Verdünnung verwendet werden. Auch solche wäßrigen Lösungen der Enzyme bzw. Enzymgemische enthalten in der Regel noch einen geringen Anteil herstellungsbedingter Begleitstoffe neben der eigentlichen Enzymaktivität bzw. neben den verschiedenen Enzymaktivitäten bei Enzymgemischen. Enzymgemische können einerseits direkt durch Fermentation erhalten werden, wobei dann die jeweils üblicherweise durch den eingesetzten Mikroorganismus gebildeten Enzyme in natürlichen Mengenverhältnissen miteinander vermischt vorliegen. Enzymgemische können andererseits jedoch auch durch einfaches Mischen von handelsüblichen Einzelenzymen hergestellt werden.

Als Granulierhilfsstoffe können im erfindungsgemäßen Verfahren enzymverträgliche und ernährungsphysiolgisch unbedenkliche Bindemittel, Füllstoffe und/oder organische Lösungsmittel eingesetzt werden. Zweckmäßige Bindemittel sind insbesondere abgebaute lösliche Stärke und/oder Weizenkleber.

In einer besonderen Ausführungsart des erfindungsgemäßen Verfahrens werden die in pulvriger Form vorgemischt vorliegenden Bestandteile für das Enzym-Vorgranulat ("Vormischung") satzweise oder kontinuierlich dem Schnellmischer zugeführt und dort ebenfalls satzweise oder kontinuierlich eine zur Einstellung des Feuchtegehaltes geeignete Menge Wasser oder eine wäßrige Lösung, ggf. mit darin gelösten Granulierhilfsstoffen (z.B. Bindemittel) und/oder darin gelöstem Enzym oder Enzymgemisch, zudosiert und dann das Enzym-Feuchtgranulat durch intensives Mischen gebildet und nach vorgegebener Verweildauer aus dem Schnellmischer entnommen bzw. kontinulierlich abgezogen.

Die Erfindung betrifft auch die nach dem erfindungsgemäßen Verfahren hergestellten Enzym-Vorgranulate, die sich in besonderer Weise zur Einarbeitung in die Teilchen eines Tierfuttermittel-Granulates eignen. Solche erfindungsgemäßen Enzymgranulate sind insbesondere weiterhin dadurch gekennzeichnet, daß sie aus
0,08 bis 22 Gew.-% (Trockensubstanz) Enzym oder Enzymgemisch, 55 bis 96,92 Gew.-% (Trockensubstanz ohne Feuchte) einer Mehltype mit einem Ausmahlungsgrad von 30 % bis 100 %, wobei die Mehlytype durch Ausmahlen einer , gegebenenfalls zuvor gewaschenen und/oder gereinigten, mit trockenem Heißdampf behandelten Mehlquelle gewonnen wurde,
gegebenenfalls bis zu insgesamt 18,5 Gew.-% Granulierhilfsstoffe (als wasserfreie Substanz berechnet), und
3 bis 12 Gew.-% Feuchte bestehen,
wobei die Summe der Bestandteile aus Enzym oder Enzymgemisch, Mehl-Trockensubstanz Feuchte und gegebenenfalls Granulierhilfsstoff 100 Gew.-% beträgt.

Zweckmäßige erfindungsgemäße Enzym-Vorgranulate bestehen aus
0,08 bis 11 Gew.-% (Trockensubstanz) Enzym oder Enzymgemisch, 66 bis 96,92 Gew.-% (Trockensubstanz ohne Feuchte) einer Mehltype mit einem Ausmahlungsgrad von 30 % bis 100 %, wobei die Mehlytype durch Ausmahlen mit trockenem Heißdampf behandelten Mehlquelle gewonnen wurde,
gegebenenfalls bis zu insgesamt 14,5 Gew.-% Granulierhilfsstoffe (als wasserfreie Substanz berechnet),
3 bis 12 Gew.-% Feuchte, wobei die Summe der Bestandteile aus Enzym oder Enzymgemisch, Mehl-Trockensubstanz Feuchte und gegebenenfalls Granulierhilfsscoff 100 Gew.-% beträgt.

Besonders bevorzugte erfindungsgemäße Enzym-Vorgranulate bestehen aus
1,9 bis 7,8 Gew.-% (Trockensubstanz) Enzym oder Enzymgemisch, 76 bis 94,6 Gew.-% (Trockensubstanz ohne Feuchte) einer Mehltype mit einem Ausmahlungsgrad von 30 % bis 100 %, wobei die Mehlytype durch Ausmahlen mit trockenem Heißdampf behandelten Mehlquelle gewonnen wurde,
insgesamt 0,5 bis 5,4 Gew.-% Granulierhilfsstoffe (als wasserfreie Substanz berechnet),
3 bis 12 Gew.-% Feuchte, wobei die Summe der Bestandteile aus Enzym oder Enzymgemisch, Mehl-Trockensubstanz Feuchte und gegebenenfalls Granulierhilfsstoff 100 Gew.-% beträgt.

Durch das erfindungsgemäße Verfahren werden vorteilhafte, aktivitätsstabile Enzym-Vorgranulate zur Einarbeitung in die Teilchen eines Tierfuttermittel-Granulates bereitgestellt. Das derart bereitgestellte erfindungsgemäße Enzym-Vorgranulat weist im Hinblick auf die Weiterverarbeitung, d.h. im Hinblick auf die Einarbeitung in die Teilchen des Tierfuttermittel-Granulates verschiedene Vorteile auf. Zum einen weist das erfindungsgemäße Enzym-Vorgranulat eine außergewöhnliche Thermostabilität, Druckstabilität und Friktionsstabilität auf. Dadurch gelingt es, das Enzym-Vorgranulat in die Teilchen eines Tierfuttermittel-Granulates im Rahmen üblicher Extrusionsverfahren einzuarbeiten, ohne daß bei den Schritten der Konditionierung (Bedüsen mit heißem Dampf) und nachfolgender Extrusion (Druckund Friktionsbeanspruchung) zur Herstellung von Strangschnittlingen wesentliche Aktivitätsverluste auftreten. Durch das erfindungsgemäße Enzym-Vorgranulat werden die Enzyme daher in einer Form zur Verfügung gestellt, die es ihnen ermöglicht, die hohe Beanspruchung in den Verfahrensschritten Konditionierung und Extrusion bei der Tierfuttermittel-Granulat-Herstellung zu überstehen. Die Erfindung betrifft daher weiterhin die Verwendung der erfindungsgemäß hergestellten Enzymvorgranulate zur Herstellung von Tierfuttermittel-Granulaten, insbesondere zur Herstellung von solchen Tierfuttermittel-Granulaten, bei denen das Enzym möglichst homogen und geschützt gegen Inaktivierung durch Extrusionsverfahren in die Teilchen des Tierfuttermittel-Granulates (d.h. sogenannte Tierfuttermittel-"Pellets") eingearbeitet werden sollen.

Neben der guten Belastbarkeit bei der Herstellung von Tierfuttermittel-Granulaten (Thermo-, Druck- und Friktionsstabilität) weist das erfindungsgemäße Enzym-Vorgranulat eine Reihe weiterer günstiger Eigenschaften auf. So zeigen die erfindungsgemäßen Enzym-Vorgranulate eine hervorragende Lagerstabilität und weisen insbesondere eine allenfalls vernachlässigbare, außerordentlich geringe Keimbelastung auf. Sie sind freifließend und zeigen daher eine gute Riesel- und Dosierfähigkeit. Darüber hinaus weisen sie nach den in der Futtermittelindustrie üblichern Testverfahren keine Verbackungsneigung auf. Im Hinblick auf die in der Futtermittelindustrie üblichen Testverfahren zur Staubbestimmung wird auch keine Neigung zur Staubbildung beobachtet. Das erfindungsgemäße Enzym-Vorgranulat besitzt weiterhin eine vorteilhafte Korngrößeneinstellung, wodurch insbesondere auch eine günstige Einmischbarkeit und Einarbeitbarkeit in die Rezepturbestandteile von Tierfuttermittel-Granulaten gewährleistet wird; die erfindungsgemäßen Enzym-Vorgranulat-Teilchen zeigen dabei keine Neigung zur Entmischung und können daher gut im Extrusionsverfahren mit den Futtermittelbestandteilen gemischt und gut im Futtermittel-Granulat eingearbeitet (dispergiert) werden.

Die nachfolgenden Beispiele sollen die vorstehende Erfindung näher erläutern, ohne diese jedoch in ihrem Umfange einzuschränken.

### Beispiel 1

### Mehlgewinnung (Heißdampfbehandlung und Ausmahlen)

Die Heißdampfbehandlung der Mehlquellen (ganze Getreidekörner oder Leguminosenfrüchte) erfolgte in einer Entkeimungsanlage mit folgendem Aufbau:
- dampfbeheizte Vorwärmschnecke, Temperatur ca 40 bis 50 °C;
- wärmeisolierter und kontinuierlich arbeitender Bedämpfer (vertikal stehender konischer Zylinder mit Höhe 5 m; Durchmesser oben ca. 40 cm, unten ca. 60 cm; Temperatur ca. 100 bis 110 °C) ;
- drei Dampf-Ringdüsen im oberen Bereich des Bedämpfers und im unteren Bereich drei vertikal angeordnete Dampflanzen;
- dampfbeheizte Austragsschnecke;
- ein nachgeschalteter Fließbett-Trockner und ein sich daran anschließender Fließbett-Kühler.

Die Getreide- oder Leguminosenkörner wurden kontinuierlich mittels der dampfbeheizten Vorwärmschnecke in den konischen Bedämpfer gefördert. Dort erfolgte über die drei Ringdüsen und drei Dampflanzen die Beaufschlagung mit trockenem Heißdampf (von 8 bar auf 0,8 bar Überdruck reduziert). Die Guttemperatur im Bedämpfer betrug ca. 100 °C, die Verweilzeit ca. 40 Minuten. Der Austrag der behandelten Getreide- oder Leguminosenkömer erfolgte über eine dampfbeheizte Schnecke, durch die das behandelte Gut zur Entfernung von Dampf und gegebenenfalls bei der Behandlung gebildeten Kondensates in einen Fließbett-Trockner überführt wurde. Nach Abkühlung in einem sich anschließenden Kühlfließbett erfolgte die Vermahlung der behandelten Getreideoder Leguminosenkörner in an sich klassischer Weise bis zum gewünschten Ausmahlungsgrad.

Die nach Heißdampf-Behandlung erhaltenen Mehle wiesen folgende durchschnittliche Eigenschaften auf:
Feuchtigkeit ca. 10 bis 15 Gew.-% (+/- 2 Gew.-%);
Gesamtkeimzahl unter 100/g;
25 g Proben waren in Bezug auf E.Coli, Salmonella, Pseudomonas aeruginosa negativ; ebenso konnte Hefe und Schimmel nicht nachgewiesen werden.

Die erfindungsgemäß heißdampfbehandelten Mehle wiesen somit eine hervorragende mikrobiologische Reinheit auf. Diese hohe mikrobilogische Reinheit wurde auch bei hohen Ausmahlungsgraden (hoher Schalenanteil im Mehl) eingehalten. Die erfindungsgemäß behandelten Mehle eigneten sich hervorragend für die nachfolgende Granulierung von Futtermittelenzymen unter milden Bedingungen insbesondere unter Bedingungen ohne thermische Behandlung bzw. thermische Keimzahlreduzierung.

### Beispiel 2

### Herstellung erfindungsgemäßer Enzym-Vorgranulate

Zur Herstellung erfindungsgemäßer Enzym-Vorgranulate für die Einarbeitung in Futtermittel-Granulate wurden Enzympräprate und gemäß Beispiel 1 erhaltene Getreide- und/oder Leguminosenmehle durch Agglomeration einer puverförmigen Ausgangsmischung unter Zusatz von Granulierflüssigkeit hergestellt. Die pulverförmige Ausgangsmischung aus Enzympräparat und Getreide- bzw. Leguminosenmehl wurde in einem kontinuierlich arbeitenden Schnellmischer/Agglomerator vom Typ Flexomix (der Firma Schugi) unter Einsprühen der Granulierflüssigkeit intensiv durchmischt und das entstandene Granulat anschließend in einem kontinuierlichen Fließbett-Trockner getrocknet. Unterkorn (< 100 µm) wurde im Fließbett-Trockner ausgeblasen (Windsichtung), Überkorn (> 800 µm) wurde abgesiebt und gemahlen. Das Fehlkorn wurde vollständig in das Granulierverfahren recycliert.

Als Enzympräparat wurden jeweils ein handelsübliches Pentosanase-Präparat und Cellulase-Präparat eingesetzt. Bei beiden Enzym-Präparaten handelt es sich um sogenannte Enzymkonzentrat-Pulver.

| Enzym* | Pentosanase-Präparat / Aktivität** | | Cellulase-Präparat/ Aktivität** | |
|---|---|---|---|---|
| **Pentosanase** | **1.030.000 (Leitaktivität)** | **EU/g** | 160.000 | EU/g |
| beca-Glucanase | 350 | EU/g | 720 | EU/g |
| alfa-Amylase | 8.800 | EU/g | 107.000 | EU/g |
| Galactomannanase | 4.300 | EU/g | 13.300 | EU/g |
| | | | | |
| **Cellulase** | 16.000 | EU/g | **30.900 (Leitaktivität)** | **EU/g** |

| | | | | |
|---|---|---|---|---|
| * = "natürliche", d.h. aus dem Herstellungsprozeß stammende, Haupt- und Begleitenzyme | | | | |
| ** =Enzymaktivitätseinheiten nach festgelegten Standard-Bestimmungsmethoden | | | | |

Als Mehl wurde in diesem Beispiel Weizenvollkornmehl mit einem Ausmahlungsgrad von 100 % eingesetzt. Die Spezifikation der Korngrößenverteilung des eingesetzten Weizenvollkornmehls (Messung mittels Labor-Luftstrahlsieb Alpine A 200 LS) war wie folgt (durchschnittliche Werte):

| Korngröße Bereich | Gew.-%-Anteil (Spezifikation) |
|---|---|
| ≥ 300 µm | ca. 4 |
| < 300 bis ≥ 250 µm | ca. 10 |
| < 250 bis ≥ 200 µm | ca. 10 |
| < 200 bis ≥ 150 µm | ca. 15 |
| < 150 bis ≥ 100 µm | ca. 15 |
| < 100 bis ≥ 50 µm | ca. 20 |
| < 50 µm | ca. 26 |

Für die Granulation erweist es sich als vorteilhaft, wenn der Feinanteil (< 50 µm) des eingesetzten Mehles möglichst gering (z.B. insbesondere unter 30 Gew.-%) gehalten wird.

Die im Granulationsverfahren eingesetzte pulverförmige Ausgangsmischung aus Enzympräparat und Mehl bestand aus 95 Gewichtsanteilen Weizenvollkornmehl als Träger und 5 Gewichtsanteilen Enzymkonzentratpulver. Die pulverförmige Ausgangsmischung wurde mit einer wäßrigen Sprühlösung, enthaltend 4 Gew.-% gelöste modifizierte Stärke, agglomeriert. Weitere beispielhafte Verfahrensbedingungen ergeben sich aus Tabelle I, die Produkteigenschaften der dabei erfindungsgemäß erhaltenen Enzym-Vorgranulate für die Einarbeitung in Tierfuttermittel-Granulate ergeben sich aus Tabelle II.

Es wurden unter minimalen Massenverlusten (< 3 Gew.-%) Enzym-Vorgranulate mit hervorragender mikrobiologischer Qualität spezifikationsgerecht hinsichtlich Korngrößenverteilung und Aktivität mit sehr guten technologischen Granulateigenschaften hergestellt.

**Tabelle I: Verfahrensbedingungen zur Herstellung eines erfindungsgemäßen Enzym-Vorgranulates**

| | **Pentosanase Vorgranulat** | | **Cellulase Vorgranulat** | |
|---|---|---|---|---|
| | Massenstrom kg/h | Feuchte Gew.-% | Massenstrom kg/h | Feuchte Gew.-% |
| Bandwaage vor Schugimischer | 550 | 13,1 | 550 | 13,1 |
| 4% Stärkelösung | 125 | 96,0 | 115 | 96,0 |
| Auslauf Schugimischer | 675 | 29,1 | 665 | 26.3 |
| Recyclat unterkorn (Fließbett-Trockner) | 118 | 11,6 | 92 | 10,6 |
| Endprodukt | 403 | 7,9 | 443 | 7,6 |
| Massenausbeute (feuchtekorrigiert) | 98 Gew.-% | | 97 Gew.-% | |
| Produkttemperatur im Fließbett-Trockner | | | | |
| Segment 1 | 50 °C | | 52 °C | |
| Segment 2 | 48 °C | | 50 °C | |
| Segment 3 | 53 °C | | 52 °C | |
| Segment 4 | 55 °C | | 56 °C | |
| Drehzahl Flexomix | 3378 rpm* | | 3484 rpm* | |

| | | | | |
|---|---|---|---|---|
| "rpm = Umdrehung pro Minute (=UpM) | | | | |

**Tabelle II: Produkteigenschaften erfindungsgemäßer Enzymvorgranulate**

| | **Pentosanase Vorgranulat** | **Cellulase Vorgranulat** |
|---|---|---|
| Aktivität | 3160 EPU/g¹⁾ | 1264 CU/g²⁾ |
| Schüttdichte | 532 g/l | 500 g/l |
| Staubzahl | 0 * | 0 * |
| Fließfaktor | 28 * | 56 * |
| Verbackungstest (Caking) | 0 * | 0 * |

| Korngrößenverteilung Endprodukt | | |
|---|---|---|
| > 800 µm (Überkorn) | 0 % | 0 % |
| > 500 - 800 µm | 26 % | 21 % |
| > 250 - 500 µm | 52 % | 57 % |
| 100 - 250 µm | 21 % | 22 % |
| < 100 µm (Unterkorn) | 0,2% | 0,1 % |

| Keimzahlen Endprodukt | | |
|---|---|---|
| Gesamtkeimzahl | 2000 /g | 1700 /g |
| Coliforme | < 30 /g | < 30 /g |
| E. coli | neg. in 25 g | neg. in 25 g |
| Salmonellen | neg. in 25 g | neg. in 25 g |
| Pseudomonas aeruginosa | neg. in 25 g | neg. in 25 g |
| Hefe | < 200 /g | < 200 /g |
| Schimmel | < 200 /g | < 200 /g |

| | | |
|---|---|---|
| * Kriterien bzgl. technologischer Granuliereigenschaften (Messung nach Standard-Methoden): Staubzahl 0-2: Fließfaktor >10: Verbackungstest <5: staubfrei frei fließend geringe Verbackungsneigung ¹⁾ EPU = Aktivität, die eine relative Fluiditätsänderung von 1 in einer Minute in einem definierten Haferschalendextran ergibt. ²⁾ CU = Aktivität, die eine relative Fluiditätsänderung von 1 in 5 Minuten in einem Carboxymethylcellulose-Substrat ergibt. | | |

### Beispiel 3

### Einarbeitung des Enzym-Vorgranulates in Teilchen eines Tierfuttermittel-Granulates (Tierfuttermittel-Pellets)

Die im Beispiel 2 erfindungsgemäß hergestellten Enzym-Vorgranulate wurden in einer Granulieranlage ("Pelletier"-Anlage) anwendungsnah in eine Futtermittelvormischung eingemischt, thermisch konditioniert und anschließend im Extrusionsverfahren granuliert ("pelletiert").

Die eingesetzte, typische Anlage zur Mischfutterpelletierung umfaßte einen Vormischer zur Mischung der festen Komponenten, einem Dosierbehälter, einen Kurzzeitkonditioneur zur Einstellung von ca. 3 % Sattdampf (Produkttemperatur 60 bis 70 °C), eine Pelletierpresse (Produkttemperatur 65 bis 80 °C) und ein Abkühlsieb.

Die Zusammensetzung der Futtermittel-Vormischungen, in die die erfindungsgemäßen Enzym-Vorgranulate (mit Pentosanase oder Cellulase) eingearbeitet wurden, ist in Tabelle III angegeben. Die Meß- und Analysendaten der Pelletierversuche mit der gegebenen Futtermittel-Vormischung und erfindungsgemäßem Vorgranulat sind in Tabelle IV wiedergegeben. Bei dem angegebenen Temperaturprofil mit einem Maximum von 73 °C bei Pentosanase-Vorgranulat und 81 °C bei Cellulase-Vorgranulat erkennt man sehr hohe Restaktivitäten nach Pelletierung, nämlich von 91% bzw. 94% der eingesetzten Enzymaktivität; derartige hohe Enzymaktivitäten werden bei Einsatz von Enzymen gemäß Stand der Technik nicht erhalten (siehe Ergebnisse von Vergleichsversuchen für Enzym-Präparate gemäß Stand der Technik in Tabelle IV).

**Tabelle III: Futtermittelvormischungen für Granulier- bzw. Pelletierversuche mit erfindungsgemäßen Enzym-Vorgranulaten**

| **Futtermittel-Komponenten** | **für Futtermittel-Granulate mit** | |
|---|---|---|
| | **Pentosanase** | **Cellulase** |
| Weizen | 37,1 Gew.-% | 24,8 Gew.-% |
| Gerste | - | 33,6 Gew.-% |
| Mais | 18,9 Gew.-% | - |
| Sojaschrot 44% | 14,5 Gew.-% | 13,4 Gew.-% |
| Erbsen | 12,6 Gew.-% | 13.2 Gew.-% |
| Tiermehl 55 | 8,4 Gew.-% | 9.1 Gew.-% |
| Tierfett | 3,2 Gew.-% | - |
| Ackerbohnen | 3,1 Gew.-% | 3,5 Gew.-% |
| Fleischknochenmehl | 2,2 Gew.-% | 2,4 Gew.-% |
| | | |

| | **für Futtermittel-Granulate mit** | |
|---|---|---|
| **Inhaltsstoffe** | **Pentosanase** | **Cellulase** |
| Feuchtigkeit | 11,4 Gew.-% | 11,8 Gew.-% |
| Rohfett | 5,5 Gew.-% | 2,9 Gew.-% |
| Rohfaser | 3,7 Gew.-% | 5,8 Gew.-% |
| Rohprotein | 19,8 Gew.-% | 21,2 Gew.-% |
| Rohstärke | 41,4 Gew.-% | 37,8 Gew.-% |
| Rohasche | 6,6 Gew.-% | 6,0 Gew.-% |
| | | |

| | **für Futtermittel-Granulate mit** | |
|---|---|---|
| **Physikalische Eingenschaften** | **Pentosanase** | **Cellulase** |
| Dichte | 1,34 g/cm³ | 1,38 g/cm³ |
| Schüttdichte | 0. 69 g/cm³ | 0, 64 g/cm³ |
| Rütteldichte | 0,74 g/cm³ | 0,60 g/cm³ |
| Schüttwinkel | 39.0 ° | 42,0 ° |
| Partikelgröße x₅₀ | 0,71 mm | 0,58 mm |

**Tabelle IV: Meß- und Analysendaten der Pelletierversuche mit Pentosanase bzw. Cellulase (jeweils als erfindungsgemäßes Enzym-Vorgranulat) und Enzymen des Standes der Technik Vorgranulat) und**

| | T | Feuchte | Aktivität | relative Aktivität | T | Feuchte | Aktivität | relative Aktivität |
|---|---|---|---|---|---|---|---|---|
| | °C | Gew-% | EPU/kg* | % | °C | Gew.-% | EPU/kg* | % |
| **Futtermittel-Granulat mit** | | | | | | | | |
| **erfindungsgemäß** | **Pentosanase** | | | | **Cellulase** | | | |
| vor Kurzzeitkonditioneur | 17 | 11,4 | 1790 | 100 | 20 | 12,3 | 476 | 100 |
| nach Kurzzeitkondioneur | 65 | 14,4 | 1678 | 93,7 | 70 | 15,4 | 510 | 107 |
| nach Presse | 73 | 14,8 | -- | -- | 81 | 15,2 | -- | -- |
| nach Kühler | 17 | 12,5 | 1621 | 90,6 | 21 | 12,7 | 448 | 94 |

| **Futtermittel-Granulat mit** | | | | | | | | |
|---|---|---|---|---|---|---|---|---|
| **Vergleichsversuche** | **Pentosanase (gemäß Stand der Technik)** | | | | **Cellulase (gemäß Stand der Technik)** | | | |
| vor Kurzzeitkondioneur | 17 | 11,4 | 2242 | 100 | 20 | 12.3 | 495 | 100 |
| nach Kurzzeitkonditioneur | 65 | 14,4 | 1561 | 69 | 71 | 15,4 | 475 | 96 |
| nach Presse | 73 | 14,8 | -- | -- | 81 | 15.2 | -- | -- |
| nach Kühler | 17 | 12,5 | 1685 | 77 | 20 | 12,7 | 285 | 58 |

| | | | | | | | | |
|---|---|---|---|---|---|---|---|---|
| * EPU = Aktivität, die eine relative Fluiditätsänderung von 1 in einer Minute in einem definierten Haferschalendextran ergibt. | | | | | | | | |

### Beispiel 5

### Thermische Stabilität erfindungsgemäßer Enzym-Vorgranulate in einer Tierfuttermittel-Matrix (Modellversuch)

Zum Nachweis der hohen thermischen Stabilität der erfindungsgemäß hergestellten Enzym-Vorgranulate wurde in einem Modellsystem die thermische Stabilität eines erfindungsgemäßen Enzym-Vorgranulates in einer Matrix aus verschiedenen Tierfuttermitteln überprüft. Hierzu wurde ein erfindungsgemäßes Cellulase- bzw. Pentosanase-Vorgranulat mit Tierfuttermittel für Geflügel (übliche Bestandteile) vermischt. Das Enzym wurde in einer Menge von 5 Gew.-% in die Futtermittel-Mischung eingebracht.

Die Messung wurde wie folgt vorgenommen:
Glasröhrchen (10x1 cm) wurden mit 5 g der Futtermittel-Mischung befüllt und anschließend in einem thermostatiertem Wasserbad jeweils für eine bestimmte Zeit bei einer bestimmten Temperatur gehalten. Nach der Temperaturbehandlung wurde das Röhrchen sofort in einem Eiswasserbad abgekühlt. Die restliche Cellulaseaktivität wurde durch eine kolorimetrische Methode bestimmt, unter Verwendung eines Referenz-Aktivitäts-Standards. Dieser Test basierte auf der enzymatischen Hydrolyse der inneren Beta-1,4-glykosidischen Bindungen einer definierten Azurin-kreuzvernetzten Cellulose. Die wiedergefundene Aktivität in % der ursprünglich eingesetzten Aktivität ist in Tabelle V für die überprüften Enzyme Pentosanase (Tabelle Va) und Cellulase (Tabelle Vb) wiedergegeben. Hierbei erweist sich Cellulase nach einer Behandlung von über einer Stunde bei Temperaturen unterhalb 80 °C mit wiedergefundenen Aktivitäten über 95 % als sehr stabil in der Futtermittel-Mischung. Auch Pentosanase erweist sich bei Temperaturen von 83 °C und einer Testdauer von 60 Minuten mit 83 % Restaktivität als sehr temperaturstabil in der Futtermittel-Mischung. Bei sehr kurzen Zeiträumen, z.B. 5 Minuten, werden noch höhere Restaktivitäten wiedergefunden (z.B. nahezu 100 % bei 95 °C, 5 Minuten).

Vergleichbare Enzymzubereitungen des Standes der Technik erweisen sich in den Futtermittel-Zusammensetzungen als wesentlich temperaturempfindlicher.

**Tabelle Va:**

| Temperaturstabilität erfindungsgemäßer Enzym-Vorgranulate in Futtermittel-Matrix: Pentosanase als Enzym | | | |
|---|---|---|---|
| Versuch Nr. | Zeit [min] | Temperatur [°C] | Restaktivität [%] |
| 1.1 | 0 | 70 | 100 |
| 1.2 | 5 | 70 | 130 |
| 1.3 | 33 | 70 | 94 |
| 1.4 | 33 | 70 | 72 |
| 1.5 | 60 | 70 | 81 |
| 1.6 | 60 | 70 | 72 |
| 2.1 | 0 | 78 | 100 |
| 2.2 | 23 | 78 | 102 |
| 2.3 | 42 | 78 | 91 |
| 3.1 | 0 | 83 | 100 |
| 3.2 | 5 | 83 | 119 |
| 3.3 | 5 | 83 | 130 |
| 3.4 | 60 | 83 | 83 |
| 3.5 | 60 | 83 | 89 |
| 4.1 | 0 | 87 | 100 |
| 4.2 | 23 | 87 | 96 |
| 4.3 | 42 | 87 | 89 |
| 5.1 | 0 | 95 | 100 |
| 5.2 | 5 | 95 | 111 |
| 5.3 | 5 | 95 | 120 |
| 5.4 | 23 | 95 | 75 |
| 5.5 | 33 | 95 | 60 |
| 5.6 | 60 | 95 | 47 |

**Tabelle Vb:**

| Temperaturstabilität erfindungsgemäßer Enzym-Vorgranulate in Futtermittel-Matrix: Cellulase als Enzym | | | |
|---|---|---|---|
| Versuch Nr. | Zeit [min] | Temperatur [°C] | Restaktivität [%] |
| 1.1 | 0 | 70 | 100 |
| 1.2 | 5 | 70 | 110 |
| 1.3 | 33 | 70 | 98 |
| 1.4 | 33 | 70 | 98 |
| 1.5 | 60 | 70 | 94 |
| 1.6 | 60 | 70 | 103 |
| 2.1 | 0 | 78 | 100 |
| 2.2 | 23 | 78 | 97 |
| 2.3 | 42 | 78 | 93 |
| 3.1 | 0 | 83 | 100 |
| 3.2 | 5 | 83 | 102 |
| 3.3 | 5 | 83 | 105 |
| 3.4 | 60 | 83 | 91 |
| 3.5 | 60 | 83 | 86 |
| 4.1 | 0 | 87 | 100 |
| 4.2 | 23 | 87 | 88 |
| 4.3 | 42 | 87 | 87 |
| 5.1 | 0 | 95.5 | 100 |
| 5.2 | 5 | 95.5 | 96 |
| 5.3 | 5 | 95.5 | 99 |
| 5.4 | 23 | 95.5 | 91 |
| 5.5 | 33 | 95.5 | 71 |
| 5.6 | 60 | 95.5 | 65 |

Soweit in der vorliegenden Anmeldung auf Enzymaktivitäten Bezug genommen wird, erfolgte die Aktivitätsbestimmung für des jeweilige Enzym nach an sich üblichen, dem Fachmann geläufigen Standardmethoden.

## Patentansprüche

1. Verfahren zur Herstellung eines aktivitätsstabilen Enzym-Vorgranulates zur Einarbeitung in Teilchen eines Tierfuttermittel-Granulates, **dadurch gekennzeichnet, dass** das Verfahren die folgenden Schritte a-e umfasst:
a) eine Mehlquelle wird mit trockenem Heißdampf behandelt,
b) diese Mehlquelle wird gemahlen, bis eine organische Mehltype mit einem Ausmahlungsgrad von 30 bis 100 Gew.% erhalten wird,
c) 80 bis 99,99 Gew.-Teile (inklusive Feuchtegehalt) der organischen Mehltype werden mit 0,01 bis 20 Gew.-Teilen eines Enzyms oder Enzymgemisches (berechnet als Trockensubstanzgehalt des eingesetzten Enzympräperates) gemischt, wobei sich die Gew.-Teile des Enzyms oder Enzymgemisches und der Mehltype auf 100 Gew.-Teile summieren,
d) diese Mischung wird durch Zugabe von Wasser oder einer wässrigen Lösung eines Enzyms oder Enzymgemisches und bis zu 20 Gew.-% Granulierhilfsstoffen (berechnet als wasserfreie Granulierhilfsstoffe) auf einen Gesamtfeuchtegehalt von 20 bis 50 Gew.-% eingestellt und in einem Schnellmischer durch intensives Mischen zu einem klebefreien Feuchtgranulat mit gewünschtem Korngrößenbereich aufgebaut,
e) das derart erhaltene Feuchtgranulat wird getrocknet.

2. Verfahren nach Anspruch 1, **dadurch gekennzeichnet, dass** zur Gewinnung der organischen Mehltype als Mehlquelle Getreidekömer, I-eguminosenfrüchte und/oder Früchte der Familie Malvaceae eingesetzt werden.

3. Verfahren nach Anspruch 1, **dadurch gekennzeichnet, dass** im Schritt a) die Behandlung der Mehlquelle mit trockenem Heißdampf mit einer Temperatur von insbesondere 100 bis etwa 110°C unter annähernd Normaldruck bis leichtem Überdruck und einer Behandlungsdauer von bis zu etwa 1 Stunde durchgeführt wurde.

4. Verfahren nach Anspruch 1, **dadurch gekennzeichnet, dass** in Schritt b) der Ausmahlungsgrad der Mehltype vorzugsweise 50 bis 100%, besonders bevorzugt 70 bis 100% beträgt.

5. Verfahren nach Anspruch 1, **dadurch gekennzeichnet, dass** man zur Herstellung des Feuchtgranulates im Schritt c) 0,01 bis 10 Gew.-Teile Enzym oder Enzymgemisch, vorzugsweise 2 bis 7 Gew.-Teile Enzym oder Enzymgemisch, 90 bis 99,99 Gew.-Teile Mehltype, vorzugsweise 93 bis 98 Gew.-Teile Mehltype gewünschtenfalls im Schritt d) bis zu insgesamt 15 Gew.-Teile, vorzugweise 0,5 bis 5 Gew.-Teile Granulierhilfsstoffe und eine berechnete Menge Wasser einsetzt, die zur Einstellung eines Feuchtegehaltes von 25 bis 40 Gew.-%, vorzugsweise von 25 bis 35 Gew.-% im Feuchtgranulat ausreicht.

6. Verfahren nach Anspruch 1, **dadurch gekennzeichnet, dass** das Enzym oder Enzymgemisch in Form eines Pulvers oder einer wässrigen Lösung des Enzyms oder Enzymgemisches eingesetzt wird.

7. Verfahren nach Anspruch 1, **dadurch gekennzeichnet, dass** das Enzym oder Enyzmgemisch eine Hydrolase, vorzugsweise aus der Gruppe der Carbohydrasen, Proteasen, Lipasen oder Esterasen, oder eine Oxinitrilase, Tannase, Chitinase, Keratinase, Oxidase oder ein Gemisch dieser Enzyme ist.

8. Verfahren nach Anspruch 7, **dadurch gekennzeichnet, dass** ein Enzym mit Nebenaktivitäten oder ein Enzymgemisch eingesetzt wird.

9. Verfahren nach Anspruch 7, **dadurch gekennzeichnet, dass** die Carbohydrasen ausgewählt sind aus β-Glucanasen, Cellulasen, Amylasen, Pentosanasen, Pectinasen, Xylanasen, Arabanasen, Hermicellulasen, Galactomannasen, Polygalacturonasen, Phytasen, Glucoamylasen, α-Galactsidasen, Pullulanasen, Driselase®.

10. Verfahren nach Anspruch 7, **dadurch gekennzeichnet, dass** die Oxidase eine Glucoseoxidase oder Peroxidase ist.

11. Verfahren nach Anspruch 7, **dadurch gekennzeichnet, dass** das Enzym Lysozym oder Muramidase ist.

12. Verfahren nach Anspruch 1, **dadurch gekennzeichnet, dass** als Granulierhilfsstoffe enzym- und ernährungsphysiologisch verträgliche Bindemittel, Füllstoffe und/oder organische Lösungsmittel (natürlichen Ursprungs) eingesetzt werden.

13. Verfahren nach Anspruch 12, **dadurch** gekennzeichent, dass als Bindemittel abgebaute lösliche Stärke und/oder Weizenkleber eingesetzt wird.

14. Verfahren nach Anspruch 1, **dadurch gekennzeichnet, dass** man ein Feuchtgranulat mit einem Korngrößenbereich von 50 bis 800 µm, vorzugsweise von 100 bis 800 µm, inbesondere von 100 bis 500 µm aufbaut.

15. Verfahren nach Anspruch 1, **dadurch gekennzeichnet, dass** man die in pulvriger Form vorgemischt vorliegenden Bestandteile für das Enzym-Granulat satzweise oder kontinuierlich in den Schnellmischer zuführt und dort ebenfalls satzweise oder kontinuierlich eine zur Einstellung des Feuchtegehaltes geeignete Menge Wasser oder eine wässrige Lösung, gegebenenfalls mit darin gelösten Granulierhilfsstoffen oder darin gelöstem Enzym oder Enzymgemisch zudosiert und man nach vorgegebener Verweildauer das Enzym-Feuchtgranulat aus dem Schnellmischer entnimmt oder kontinuierlich abzieht.

16. Aktivitätsstabiles Enzym-Vorgranulat zur Einarbeitung in Teilchen eines Tierfuttermittel-Granulates, **dadurch gekennzeichnet, dass** das Enzym-Vorgranulat nach einem Verfahren der Ansprüche 1 bis 15 erhältlich ist.

17. Enzym-Vorgranulat nach Anspruch 16, **dadurch gekennzeichnet**, das es aus 0,08 bis 22 Gew.-% (Trockensubstanz) des Enzyms oder Enzymgemisches, 55 bis 96,92 Gew.-% (Trockensubstanz) der organischen Mehltype mit einem Ausmahlungsgrad von 30% bis 100%, wobei die Mehltype durch Ausmahlen einer mit trockenem Heißdampf behandelten Mehlquelle gewonnen wurde, gegebenenfalls bis zu insgesamt 18,5 Gew.-% Granulierhilfsstoffen (als wasserfreie Substanz berechnet) mit 3 bis 12 Gew.-% Feuchte besteht, wobei die Summe der Bestandteile aus Enzym oder Enzymgemisch, Mehl-Trockensubstanz, Feuchte und gegebenenfalls Granulierhilfsstoff 100 Gew.-% beträgt.

18. Verwendung von Enzym-Vorgranulaten, gemäß einer der Ansprüche 16 oder 17 zur Herstellung von Tierfuttermittel-Granulaten, vorzugsweise derart, dass die Enzym-Vorgranulate in übliche Tierfuttermittelbestandteile eingemischt und diese Mischung durch Extrusion zu einem homogenen Tierfuttermittel-Granulat verarbeitet wird.

## Claims

1. Process for producing stable-activity enzyme pregranules for incorporation into particles of animal feed granules, **characterized in that** the process comprises the following steps a-e:
a) a flour source is treated with dry superheated steam,
b) this flour source is ground until an organic flour type having an extraction rate of 30 to 100% by weight is obtained,
c) 80 to 99.99 parts by weight (including moisture content) of the organic flour type are mixed with 0.01 to 20 parts by weight of an enzyme or enzyme mixture (calculated as dry matter content of the enzyme preparation used), wherein the parts by weight of the enzyme or enzyme mixture and of the flour type total 100 parts by weight,
d) this mixture, by addition of water or an aqueous solution of an enzyme or enzyme mixture and up to 20% by weight granulation aids (calculated as anhydrous granulation aids), is set to a total moisture content of 20 to 50% by weight and, in a high-speed mixer, is made up by intensive mixing to give non-sticky moist granules having a desired particle size range,
e) the moist granules thus obtained are dried.

2. Process according to Claim 1, **characterized in that** to obtain the organic flour type, as flour source, use is made of cereal grains, legumine fruits and/or fruits of the family Malvaceae.

3. Process according to Claim 1, **characterized in that**, in step a), the flour source was treated with dry superheated steam at a temperature of, in particular, 100 to about 110°C, under approximately atmospheric pressure to slight superatmospheric pressure, and a treatment time of up to about 1 hour.

4. Process according to Claim 1, **characterized in that**, in step b), the extraction rate of the flour type is preferably 50 to 100%, particularly preferably 70 to 100%.

5. Process according to Claim 1, **characterized in that**, to produce the moist granules, in step c), use is made of 0.01 to 10 parts by weight of enzyme or enzyme mixture, preferably 2 to 7 parts by weight of enzyme or enzyme mixture, 90 to 99.99 parts by weight of flour type, preferably 93 to 98 parts by weight of flour type, if desired, in step d), up to in total 15 parts by weight, preferably 0.5 to 5 parts by weight, of granulation aids and a calculated amount of water which is sufficient to set a moisture content of 25 to 40% by weight, preferably 25 to 35% by weight, in the moist granules.

6. Process according to Claim 1, **characterized in that** the enzyme or enzyme mixture is used in the form of a powder or an aqueous solution of the enzyme or enzyme mixture.

7. Process according to Claim 1, **characterized in that** the enzyme or enzyme mixture is a hydrolase, preferably from the group of carbohydrases, proteases, lipases or esterases, or an oxynitrilase, tannase, chitinase, keratinase, oxidase or a mixture of these enzymes.

8. Process according to Claim 7, **characterized in that** use is made of an enzyme having side activities, or of an enzyme mixture.

9. Process according to Claim 7, **characterized in that** the carbohydrases are selected from β-glucanases, cellulases, amylases, pentosanases, pectinases, xylanases, arabanases, hemicellulases, galactomannases, polygalacturonases, phytases, glucoamylases, α-galactsidases, pullulanases, Driselase^{®}.

10. Process according to Claim 7, **characterized in that** the oxidase is a glucose oxidase or peroxidase.

11. Process according to Claim 7, **characterized in that** the enzyme is lysozyme or muramidase.

12. Process according to Claim 1, **characterized in that**, as granulation aids, use is made of enzymaticly and nutritionally acceptable binders, fillers and/or organic solvents (of natural origin).

13. Process according to Claim 12, **characterized in that** the binder used is degraded soluble starch and/or wheat gluten.

14. Process according to Claim 1, **characterized in that** moist granules are made up having a particle size range of 50 to 80 µm, preferably of 100 to 800 µm, in particular of 100 to 500 µm.

15. Process according to Claim 1, **characterized in that** the components for the enzyme granules which are present premixed in pulverulent form are fed into the high-speed mixer batchwise or continuously, and there, likewise batchwise or continuously, an amount of water or an aqueous solution, which is suitable for adjusting the moisture content, if appropriate having granulation aids dissolved therein or enzyme or enzyme mixture dissolved therein is added, and after a predetermined dwell time, the enzyme-moist granules are taken from the high-speed mixer, or withdrawn continuously.

16. Stable- activity enzyme pregranules for incorporation into particles of animal feed granules, **characterized in that** the enzyme pregranules are obtainable by a process of Claims 1 to 15.

17. Enzyme pregranules according to Claim 16, **characterized in that** they comprise 0.08 to 22% by weight (dry matter) of the enzyme or enzyme mixture, 55 to 96.92% by weight (dry matter) of the organic flour type having an extraction rate of 30% to 100%, wherein the flour type was obtained by milling a flour source treated with dry superheated steam, if appropriate up to in total 18.5% by weight of granulation aids (calculated as anhydrous substance) with 3 to 12% by weight of moisture, wherein the total of the components of enzyme or enzyme mixture, flour dry matter, moisture and if appropriate granulation aid is 100% by weight.

18. Use of enzyme pregranules according to one of Claims 16 or 17 for producing animal feed granules, preferably in such a manner that the enzyme pregranules are mixed into conventional animal feed components and this mixture is processed by extrusion to form homogeneous animal feed granules.

## Revendications

1. Procédé de fabrication d'un prégranulé enzymatique à activité stable pour incorporation dans des particules d'un granulé d'un produit pour l'alimentation animale, **caractérisé en ce que** le procédé comprend les étapes a-e ci-après :
a) on traite une source de farine avec de la vapeur surchauffée sèche,
b) on broie cette source de farine jusqu'à obtention d'un type de farine organique ayant un taux d'extraction de 30 à 100 % en poids,
c) on mélange 80 à 99,99 parties en poids (y compris l'humidité contenue) du type de farine organique à 0,01 à 20 parties en poids d'une enzyme ou d'un mélange d'enzymes (exprimé par la teneur en extrait sec de la préparation enzymatique utilisée), la somme des parties en poids de l'enzyme ou du mélange d'enzyme et du type de farine faisant 100 parties en poids,
d) on ajuste à une valeur de 20 à 50 % en poids la teneur totale en humidité de ce mélange, par addition d'eau ou d'une solution aqueuse d'une enzyme ou d'un mélange d'enzymes, et d'une quantité allant jusqu'à 20 % en poids d'auxiliaires de granulation (exprimés en auxiliaires de granulation anhydres), et, dans un mélangeur à grande vitesse, on crée à partir de ce mélange, par mélange intense, un granulé humide non adhérent présentant la plage granulométrique souhaitée,
e) on sèche le granulé humide ainsi obtenu.

2. Procédé selon la revendication 1, **caractérisé en ce que**, pour obtenir le type de farine organique, on utilise en tant que source de farine des grains de céréales, des fruits de légumineuses et/ou des fruits de la famille des malvacées.

3. Procédé selon la revendication 1, **caractérisé en ce que**, dans l'étape a), le traitement de la source de farine avec de la vapeur surchauffée sèche à une température en particulier de 100 à environ 110°C est mis en oeuvre sous une pression comprise entre une pression approximativement normale et une légère surpression, pour une durée du traitement allant jusqu'à environ 1 heure.

4. Procédé selon la revendication 1, **caractérisé en ce que**, dans l'étape b), le taux d'extraction du type de farine est de préférence de 50 à 100 %, d'une manière particulièrement préférée de 70 à 100 %.

5. Procédé selon la revendication 1, **caractérisé en ce que**, pour fabriquer le granulé humide, on utilise dans l'étape c) 0,01 à 10 parties en poids de l'enzyme ou du mélange d'enzymes, de préférence 2 à 7 parties en poids de l'enzyme ou du mélange d'enzymes, 90 à 99,99 parties en poids du type de farine, de préférence 93 à 98 parties en poids du type de farine, éventuellement dans l'étape d) jusqu'à un total de 15 parties en poids, de préférence de 0,5 à 5 parties en poids d'auxiliaires de granulation, et une quantité calculée d'eau qui suffit pour ajuster la teneur en humidité du granulé humide à une valeur de 25 à 40 % en poids, de préférence de 25 à 35 % en poids.

6. Procédé selon la revendication 1, **caractérisé en ce que** l'enzyme ou le mélange d'enzymes est utilisé sous forme d'une poudre ou d'une solution aqueuse de l'enzyme ou du mélange d'enzymes.

7. Procédé selon la revendication 1, **caractérisé en ce que** l'enzyme ou le mélange d'enzymes est une hydrolase, de préférence du groupe des carbohydrases, des protéases, des lipases ou des estérases, ou une oxynitrilase, une tannase, une chitinase, une kératinase, une oxydase ou un mélange de ces enzymes.

8. Procédé selon la revendication 7, **caractérisé en ce qu'**on utilise une enzyme ou un mélange d'enzymes ayant des activité secondaires.

9. Procédé selon la revendication 7, **caractérisé en ce que** les carbohydrases sont choisies parmi les β-glucanases, les cellulases, les amylases, les pentosanases, les pectinases, les xylanases, les arabanases, les hémicellulases, les galactomannases, les polygalacturonases, les phytases, les glucoamylases, les α-galactosidases, les pullulanases, la Driselase®.

10. Procédé selon la revendication 7, **caractérisé en ce que** l'oxydase est une glucose-oxydase ou une peroxydase.

11. Procédé selon la revendication 7, **caractérisé en ce que** l'enzyme est le Lysozym ou la Muramidase.

12. Procédé selon la revendication 1, **caractérisé en ce qu'**on utilise en tant qu'auxiliaires de granulation, des matières de charge et/ou des solvants organiques (d'origine naturelle), compatibles du point de vue de la physiologie des enzymes et de l'alimentation.

13. Procédé selon la revendication 12, **caractérisé en ce qu'**on utilise en tant que liant de l'amidon soluble dégradé et/ou du gluten de froment.

14. Procédé selon la revendication 1, **caractérisé en ce qu'**on construit un granulé humide ayant une plage granulométrique de 50 à 800 µm, de préférence de 100 à 800 µm, en particulier de 100 à 500 µm.

15. Procédé selon la revendication 1, **caractérisé en ce qu'**on introduit dans le mélangeur à grande vitesse, d'une manière discontinue ou continue, les constituants du granulé enzymatique présents prémélangés sous forme pulvérulente, et on y introduit, d'une manière continue ou discontinue, une quantité d'eau ou d'une solution aqueuse permettant d'ajuster la teneur en humidité, éventuellement avec des auxiliaires de granulation qui y sont dissous, ou une enzyme ou un mélange d'enzymes qui y sont dissous, et, après un temps de séjour prédéterminé, on prélève le granulé enzymatique humide du mélangeur à grande vitesse, ou on l'en soutire en continu.

16. Prégranulé enzymatique à activité stable pour incorporation dans des particules d'un granulé d'un produit pour l'alimentation animale, **caractérisé en ce que** le prégranulé enzymatique peut être obtenu par un procédé selon les revendications 1 à 15.

17. Prégranulé enzymatique selon la revendication 16, **caractérisé en ce qu'**il est constitué de 0,08 à 22 % en poids (en extrait sec) de l'enzyme ou du mélange d'enzymes, de 55 à 96,92 % en poids (en extrait sec) du type de farine organique ayant un taux d'extraction de 30 à 100 %, le type de farine ayant été obtenu par extraction d'une source de farine traitée avec de la vapeur surchauffée sèche, éventuellement d'une quantité allant en tout jusqu'à 18,5 % en poids d'auxiliaires de granulation (exprimés en substance anhydre) ayant une humidité de 3 à 12 % en poids, la somme des constituants de l'enzyme ou du mélange d'enzyme, de la matière sèche de farine, de l'humidité et éventuellement de l'auxiliaire de granulation, étant de 100 % en poids.

18. Utilisation de prégranulés enzymatiques selon l'une des revendications 16 ou 17 pour fabriquer des granulés d'un produit pour l'alimentation animale, de préférence par le fait que l'on incorpore par mélange les prégranulés enzymatiques dans des constituants usuels de produits pour l'alimentation animale, et on transforme ce mélange par extrusion en un granulé homogène pour l'alimentation animale.
